# EUROPEAN PATENT SPECIFICATION

(11) **EP 2 309 966 B1**
(45) Date of publication and mention of the grant of the patent: **17.07.2013**
(21) Application number: 08779461.6
(22) Date of filing: 05.08.2008
(51) Int. Cl.: A61F 13/494, A61F 13/49, A61F 13/493, A61F 13/496

(54) **ABSORBENT ARTICLE**
SAUGFÄHIGER ARTIKEL
ARTICLE ABSORBANT

(43) Date of publication of application: 20.04.2011
(73) Proprietor: SCA Hygiene Products AB, 405 03 Göteborg (SE)
(72) Inventor: CORNELIUSSON, Helena, S-445 36 Bohus (SE)
(74) Representative: Szelagowski, Helen
(86) International application number: PCT/SE2008/050904
(87) International publication number: WO 2010/016785

(56) References cited:
- EP-A1- 0 518 044
- EP-A1- 0 587 196
- EP-A1- 1 652 500
- EP-A2- 1 034 761
- EP-A2- 1 312 327
- WO-A1-02/05739
- WO-A1-97/10785
- GB-A- 2 283 662
- US-A- 5 649 919
- US-A1- 2007 239 130

## Description

### TECHNICAL FIELD

The present invention relates to an absorbent article according to the preamble of claim 1 and to a method of manufacturing such an absorbent article.

### BACKGROUND OF THE INVENTION

Conventional absorbent articles, for example child and adult diapers, training pants, pull-up diapers, adult incontinence pants, belted products and swim pants, are provided with elasticized regions to improve user fit and comfort. It is known, for example, to provide elasticized leg openings to form a gasket about the legs of a wearer to thereby reduce the risk of leakage. It is also known to use standing-gather webs to create a "pocket" on the diaper to thereby retain faeces.

Typically, elasticized regions are created by intermittently bonding elastic threads or ribbons under tension to a substrate forming at least a part of the chassis of the absorbent article. For a diaper, typical substrates are the backsheet, topsheet and standing-gather webs. When the tension is released, the elastic threads or ribbons contract and gather the substrate. The elastic threads or ribbons may be attached as discrete elements or, particularly when manufacturing diapers in the machine direction, as continuous elements which are then severed when the diapers are separated from each other. Whilst it is desirable to provide elasticized leg openings in the crotch region of a diaper, it is undesirable to gather the lateral edges of the diaper in the waistband regions of the diaper. Accordingly, it is known to leave the ends of the elastic threads unadhered in the waistband regions of the diaper. As such, when the elastic threads are severed, the unattached ends of the elastic threads then snap back, that is retract towards the crotch region of the diaper, in channels created in the absorbent article, leaving the waistband regions of the absorbent product in a non-elasticized state. The snap-back effect is described, for example, in US Patent 4,081,301.

It is known to provide diapers with discrete side panels extending laterally from the chassis of the absorbent article. The side panels generally comprise fastening means to permit the diaper to be worn about the waist of a wearer. In order to obtain a smooth surface upon which to attach the side panels, the side panels are attached to the chassis in a non-elasticized region. One example of such an absorbent product is disclosed in WO 00/07534 in which opposed side panels are attached to the rear waistband region of a diaper. The therein-described diaper has standing-gather webs forming leg elastic channels accommodating elastic threads. To ensure that the side panel attachment regions are free from gathering, the side panels are attached to the chassis at locations laterally outwards of the leg elastic channels.

With the diaper design described above, the waistband portion must be sufficiently wide to provide an attachment area for the side panel which is non-elasticized in order to prevent gathering. The side panel is therefore attached well outside of the leg channel. Often, this means that the material making up the chassis which is laterally outside of the leg channels must be trimmed away in the crotch region to ensure a better fit. Not only is this trimming an additional manufacturing step, any removal of material means that such material is wasted.

### SUMMARY OF THE INVENTION

It is therefore an object of the present invention to provide an absorbent article such as a diaper in which less material can be used to make its chassis, but which still fits well to the wearer.

This object is achieved in accordance with the present invention by an absorbent article having a chassis comprising a backsheet and an absorbent structure. The chassis extends in a longitudinal direction about a longitudinal axis (L) dividing the chassis into a first lateral half and a second lateral half. The chassis further extends in a transverse direction about a transverse axis (T). The chassis is divided along the longitudinal axis into three regions, namely a first end region intended to be worn against the back of a user, a crotch region intended to be worn against the crotch of the user and a second end region intended to be worn against the stomach of the user. The absorbent structure is associated with the backsheet such that the first lateral half comprises a first lateral margin terminating in a first lateral edge, and the second lateral half comprises a second lateral margin terminating in a second lateral edge, with the first and said second lateral margins being free of the absorbent structure. Leg elastic material extends in the longitudinal direction in the first and second lateral margins. A first and a second standing-gather web extending in the longitudinal direction are affixed to the chassis in the first and second lateral margins. The first web forms a first leg elastic channel and the second web forms a second leg elastic channel, with the leg elastic channels accommodating the leg elastic material. A first and a second side panel are attached to the chassis in one of the first and second end regions, with the first side panel extending outwardly from the first lateral edge of the chassis and the second side panel extending outwardly from the second lateral edge of the chassis. The first leg elastic channel and the first side panel are in an overlapping relationship in a first overlap region and the second leg elastic channel and the second side panel are in an overlapping relationship in a second overlap region. The leg elastic material is at least partly adhered to the first and second standing-gather webs inside the first and second leg elastic channels at least in the crotch region such that the leg elastic material is free to move in the longitudinal direction relative to the standing-gather web inside the leg elastic channels in the overlap regions.

Since, in accordance with the present invention, the leg elastic material is free to move in the leg elastic channels and the leg elastic channels overlap the side panels, a narrow article can be attained without there being undesirable gathering of the article in the overlap regions.

In one embodiment of the invention, the first and second side panels are attached to the first end region of the chassis, i.e. the region which is intended to be worn against the back of a user.

In another embodiment, each of the first and second side panels is fastened between the backsheet and a topsheet which covers the absorbent structure.

In a further embodiment, a part of the first standing-gather web contacts the first side panel in the first overlap region and a part of the second standing-gather web contacts the second side panel in the second overlap region.

In another embodiment, the leg elastic material in the first leg elastic channel extends over at least part of the first overlap region and the leg elastic material in the second leg elastic channel extends over at least part of the second overlap region.

In a further embodiment, the first and second leg elastic channels, formed by the standing-gather web accommodating the leg elastic material, have a closed circumference at least in the first and second overlap regions. These leg elastic channels may have a closed circumference along substantially their entire length.

In another embodiment, the leg elastic material in the first and second leg elastic channels is free to move in a free-moving length (D) which is 10-30% of the length (H) of the absorbent article, preferably 12-28% and most preferably 15-25%.

To simplify production and to keep costs to a minimum, in one embodiment of the invention the first and second lateral edges of the chassis are straight and, in a further embodiment, the first and second lateral edges extend parallel to the longitudinal axis (L).

To provide increased comfort and better fit, in one embodiment of the invention the first and second side panels comprise an elastic material.

To create a suitable pocket for faeces, each of the first and second standing-gather webs further comprises a standing-gather elastic extending in the longitudinal direction.

Each of the first and second standing-gather webs further comprises a free-standing first end region which extends freely from a proximal end thereof. The proximal end is preferably located transversally inwardly of the leg elastic material.

Advantageously, the standing-gather elastic is arranged to extend along said free-standing first end region.

In another embodiment, the maximum width A of the first lateral margin is 10-70% of the width B of the first lateral half, preferably 15-65%, more preferably 20-55% and most preferably 20-40%.

The exact size of the absorbent article will depend on the size of the intended wearer. In one embodiment, the maximal width A of the first lateral margin is less than 65 mm, preferably less than 60 mm, more preferably less than 50 mm and most preferably less than 35 mm. In the same or another embodiment, the transverse distance C from the first lateral edge to the first channel is less than 50 mm, preferably less than 40 mm, more preferably less than 30 mm and most preferably less than 20 mm.

The transverse distance C from the first lateral edge to the first channel is 10-85% of the maximal width A of the first lateral margin, preferably 10-65%, more preferably 10-40% and most preferably 10-20%.

To further improve fit and comfort, in a further embodiment the end region in which the side panels are attached to the chassis further comprises a waist elastic being elastic in the transverse direction of the absorbent article, said waist elastic being located in the chassis outside of the absorbent structure.

In another embodiment, the largest transversal extension of the chassis is 0-40% larger than the smallest transversal extension of the chassis, preferably 0-20% and most preferably 0%.

The leg elastic material and/or the standing gather elastic material may be intermittently or continuously fastened.

The absorbent article of the invention may form an open diaper, which is intended to be closed when worn on an intended user. Alternatively, the side panels may be constituted by belt halves so that the absorbent article forms a belted product. In another embodiment, the absorbent article forms a pant diaper.

It is a further object of the present invention to provide a method for manufacturing the above-described types of absorbent articles.

This object is achieved by a method comprising the steps of:
- placing an absorbent structure on a backsheet such that the backsheet presents a first lateral margin terminating in a first lateral edge and a second lateral margin terminating in a second lateral edge, the first and second lateral margins being free from the absorbent structure;
- attaching a first side panel and a second side panel to the backsheet in the first and second lateral margins, respectively, such that the side panels extend outwardly from the lateral edges;
- providing a first and a second standing-gather web having a first and a second leg elastic channel, each leg elastic channel containing tensioned leg elastic material intermittently affixed to the leg elastic channels;
- attaching the first and second standing-gather webs to the first and second lateral margins such that the first and second standing-gather webs partly overlap the first and second side panels in a first and a second overlap region, the leg elastic material being unattached to the first and second leg elastic channels in the first and second overlap regions, and
- releasing the tension in the leg elastic material to allow free-moving ends of the leg elastic material to snap back along the first and second leg elastic channels.

In a further embodiment, the first and second side panels are constituted by belt halves.

### BRIEF DESCRIPTION OF THE DRAWINGS

The present invention will hereinafter be explained in greater detail by means of nonlimiting examples and with reference to the appended drawings in which:
- Fig.1: is a schematic plan view of an absorbent article according to a first embodiment of the invention in a flat-out uncontracted condition;
- Fig. 2: is a cross-sectional view along line II-II of Fig. 1, though showing leg elastic material prior to snap back;
- Fig. 3: is a partially cut away schematic plan view of a region of the absorbent article according to the invention;
- Fig. 4: is a cross-sectional view corresponding to that of Fig. 2, though for a different embodiment;
- Fig. 5: is a schematic perspective view of a part of a manufacturing process for making an absorbent article of the present invention, and
- Fig. 6: is a schematic perspective view showing how standing-gather webs are produced in the method according to the invention.

### DETAILED DESCRIPTION OF PREFERRED EMBODIMENTS

In the drawings, reference number 10 generally denotes an absorbent article according to the present invention. In the illustrated embodiment the absorbent article 10 is in the form of a diaper which is intended to be worn about the waist of a wearer.

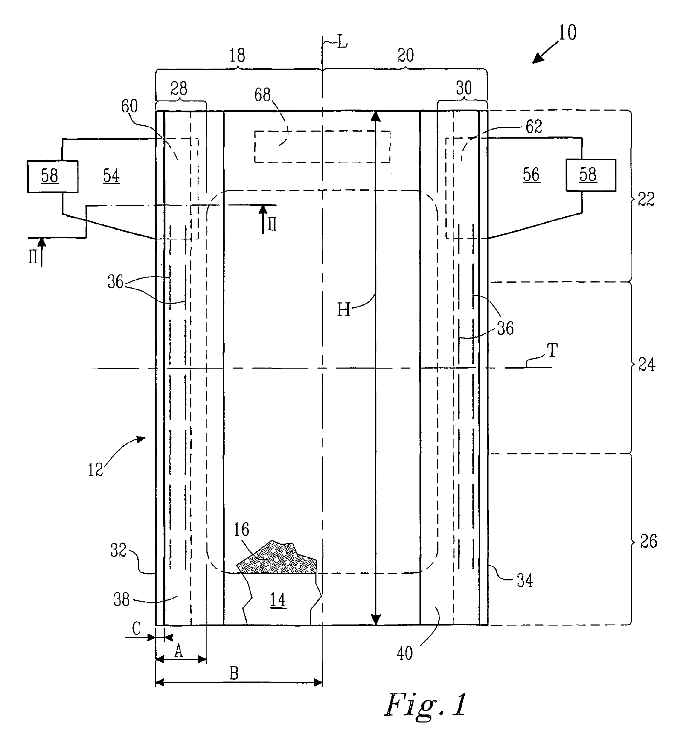

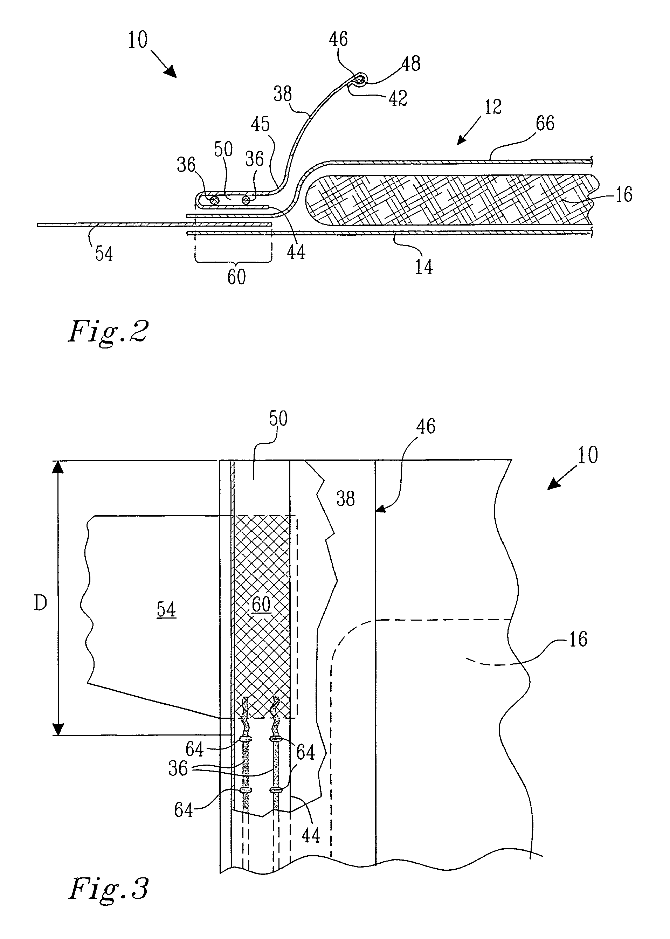

With particular reference to Figs. 1 and 3, it will be apparent that the absorbent article 10 has a chassis 12 comprising a backsheet 14 and an absorbent structure 16. The chassis extends a distance H in a longitudinal direction about a longitudinal axis L, which normally coincides with the running direction of the production machine. The absorbent structure 16 is centered transversally about the longitudinal axis L. The longitudinal axis L divides the chassis 12 into a first lateral half 18 and a second lateral half 20. The chassis further extends in a transverse direction about a transverse axis T. The chassis 12 may be divided along the longitudinal axis L in a first end region 22, a crotch region 24 and a second end region 26. The first end region 22 is intended to be worn against the back of the wearer and the second end region 26 against the stomach of the wearer when the absorbent article is worn.

The absorbent structure 16 is generally centered with respect to the longitudinal axis L on the chassis 12 such that the first lateral half 18 of the chassis presents a first lateral margin 28 that is free from the absorbent structure 16. In a corresponding manner, the second lateral half 20 presents a second lateral margin 30 that is also free from the absorbent structure. The first and second lateral margins 28, 30 terminate in first and second lateral edges 32, 34, respectively. In a manner which will be explained in greater detail below, the absorbent article 10 is provided with leg elastic material 36 extending in the longitudinal direction in the first and second lateral margins 28, 30.

The absorbent article 10 is further provided with a first standing-gather web 38 and a second standing-gather web 40 fixed to the chassis 12 in the first and second lateral margins 28, 30, respectively. In the illustrated embodiment the first and second standing-gather webs extend in the longitudinal direction over the entire length of the absorbent article. It will be appreciated, however, that, depending on production techniques employed, the standing gather webs may terminate short of the transverse edges of the chassis. As is most readily apparent from Fig. 2, the first standing-gather web 38 has a free-standing first end edge region 42 and a second end edge region 44 adjacent the chassis 12. The free-standing first end edge region 42 extends freely from a proximal end 45 thereof. The proximal end 45 is advantageously at least intermittently joined to the topsheet 66. The proximal end 45 is in this case located transversally inwardly of the leg elastic material 36.

The free-standing first end edge region 42 delimits a first pocket 46 which, in a conventional manner, accommodates a thread 48, yarn or ribbon of elastic material extending in the longitudinal direction. The second end edge region 44 delimits a first leg elastic channel 50 for the leg elastic material 36. The first leg elastic channel 50 and the first pocket 46 are formed by folding the first standing-gather web back on itself and securing the end regions in a suitable (not shown) manner, such as by adhesive or ultrasonic welding, to the adjacent standing-gather web material. In an identical manner, the second standing-gather web 40 forms a second leg elastic channel 52 for the leg elastic material 36 in the second lateral margin 36 of the absorbent article. Thus, the first and second leg elastic channels can have a closed circumference along substantially their entire length or, optionally, along only a part or parts of their length.

To permit the absorbent article to be worn around the waist of a wearer, a first side panel 54 and a second side panel 56 are attached to the chassis 12 in the first end region 22 such that the side panels extend outwardly from the lateral edges 32, 34 of the chassis 12. Although, for ease of putting the absorbent article onto the wearer, it is preferred that the side panels be located in the first end region, it is to be understood that, under certain circumstances, it may be desirable to attach the side panels in the second end region 26. In a not-shown embodiment, the absorbent article could be furnished with side panels in both end regions. The first and second side panels 54, 56 may be provided with fastening members 58 for engagement with (not shown) co-operating receiving members on the backsheet 12 in the second end region 26. Such fastening members and receiving members are of conventional type, such as adhesive tabs or hook-and-loop tabs, and will not be described in any further detail.

In accordance with the present invention, and as is most readily derivable from Fig. 3, the first leg elastic channel 50 and the first side panel 54 are in an overlapping relationship in a first overlap region 60, denoted by cross-hatching in the drawing. In an identical manner, the second leg elastic channel 52 and the second side panel 56 are in a corresponding overlapping relationship in a second overlap region 62. In addition, the leg elastic material 36 is at least partially adhered to the first and second standing-gather webs inside the first and second leg elastic channels, respectively, at least in the crotch region 24 of the chassis 12. Intermittent bonding of the leg elastic material 36 to the first standing-gather web 38 is indicated in Fig. 3 by reference number 64. Finally, so that the chassis will remain ungathered in the first and second overlap regions 60, 62, the leg elastic material 36 is not bonded to the standing-gather webs in these regions. As such, the leg elastic material 36 is free to move in the longitudinal direction relative to the standing-gather webs inside the leg channels in these regions of overlap.

In Fig. 2 an embodiment is shown with two elastic yarns 36 for the leg elastic material and one elastic yarn 48 for the standing-gather elastic material, though it is to be understood that the numbers of yarn for the leg elastic material and the standing-gather elastic material may vary.

The leg elastic material 36 and/or the standing gather elastic material 48 may be intermittently or continuously fastened. For example, continuous fastening may be achieved by use of an elastic adhesive.

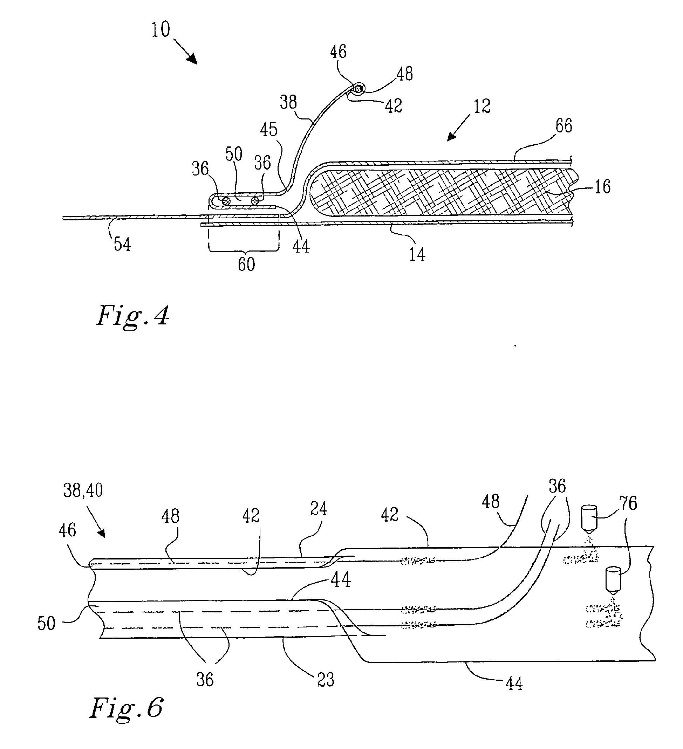

In the embodiment illustrated in Figs. 1 to 3, the absorbent article is provided with a topsheet 66 which is essentially coextensive with the backsheet and the first and second side panels 54, 56 are advantageously attached to the chassis between the backsheet 14 and the topsheet 66. In an alternative embodiment illustrated in Fig. 4, the topsheet 66 terminates short of the first and second leg elastic channels 50, 52 such that the first and second side panels are fastened between the backsheet 14 and the respective leg elastic channel 50, 52. In this manner, less topsheet material is required. In a not shown embodiment, the topsheet 66 and backsheet 14 may be coextensive, with the first and second side panels 54, 56 being attached to the chassis 12 between the topsheet and the respective leg elastic channels.

Since the various components of the absorbent article 10 are joined to each other using conventional techniques, the drawings have not been cluttered with details of how the components are united to each other.

With particular reference to Fig. 3, and as is mentioned above, each thread, yarn or ribbon of elastic leg material 36 is bonded to the standing-gather web 38 by intermittent bonding 64. The intermittent bonding can be attained by any means known in the art, such as by ultrasonic bonding, by an adhesive, by heat and/or pressure, etc. Seen in the longitudinal direction of the absorbent article starting from the first end region 22, the intermittent bonding 64 commences at an initial bonding site adjacent the side panel 54. The initial bonding site is located a distance D from the upper transverse edge of the chassis 12. During the production of articles in which the elastic leg material is tensioned so as to extend over the entire length of the chassis, the distance D corresponds to a free-moving distance of the leg elastic material, i.e. the distance over which the leg elastic material is free to snap back. The distance D is preferably 10% to 30%, more preferably 12% to 28% and most preferably 15% to 25%, of the length H of the chassis 12.

The proximity of the initial bonding site of the intermittent bonding 64 to the first and second side panels 54, 56 will dictate whether any snapped-back leg elastic material 36 extends over some of the first and second overlap regions 60, 62. In the embodiment illustrated in Fig. 3 it will be seen that the leg elastic material 36 extends over at least a part of the first overlap region 60.

In one embodiment of the invention, the first and second leg elastic channels 50, 52 formed by the first and second standing-gather webs 38, 40 have a closed circumference at least in the first and second overlap regions 60, 62.

In order to minimize material consumption, it is preferred that the absorbent article 10 has straight lateral edges 32, 34 and that these extend parallel to the longitudinal axis L. It is of course conceivable that the lateral edges be curved, though this would add complexity to the production process and lead to material loss if material has to be cut away. Accordingly, the chassis of the absorbent article 10 will have a largest transversal extension and a smallest transversal extension. When the lateral edges 32, 34 are curved, the largest transversal extension of the chassis will be 0-40% larger than the smallest transversal extension, preferably 0-20% and most preferably 0%, i.e. when the lateral edges are parallel to the longitudinal axis L.

As is shown in Fig. 1, the first lateral margin has a maximum width A in the transverse direction and the first lateral half has a width B in the same direction. In one embodiment of the invention, the maximum width A is 10-70% of the width B, preferably 15-65%, more preferably 20-55% and most preferably 20-40%. Depending on the type of absorbent article, the maximum width A of the first lateral margin 28 is less than 65 mm, preferably less than 60 mm, more preferably less than 50 mm and most preferably less than 35 mm.

The first leg elastic channel 50 may be spaced a transverse distance C from the first lateral edge 32. This distance C may be less than 50 mm, preferably less than 40 mm, more preferably less than 30 mm and most preferably less than 20 mm. As a ratio of the maximum width A of the first lateral margin 28, the transverse distance C may be 10-85% of the maximal width A, preferably 10-65%, more preferably 10-40% and most preferably 10-20%.

It will be appreciated that the above recited relative dimensions for the first lateral margin, first lateral half and first leg elastic channel also apply for the second lateral margin, second lateral half and second leg elastic channel.

In addition to the leg elastic material 36 and standing-gather elastic material 48, the absorbent article of the present invention may also include further elastic material, such as a waist elastic 68. As illustrated in Fig. 1, the waist elastic 68 may advantageously extend in the transverse direction between the first and second side panels 54, 56 in a region of the absorbent article outside of the absorbent structure 16.

The materials making up the absorbent article of the present invention may be selected from any of the materials commonly used for such products and may include environmentally friendly materials from renewable sources and/or biodegradable material. Thus, the topsheet 66 can comprise a nonwoven material, e.g. spunbond, meltblown, carded, hydroentangled, wetlaid, etc. Suitable nonwoven materials can be composed of man-made fibres, such as polyester, polyethylene, polypropylene, viscose, rayon, etc. or natural fibres, such as wood pulp or cotton fibres, or from a mixture of natural and man-made fibres. The topsheet material may further be composed of tow fibres, which may be bonded to each other in a bonding pattern, as e.g. disclosed in EP-A-1 035 818. Further examples of possible topsheet materials include porous foams, apertured plastic films, laminates of nonwoven materials and apertured nonwoven fabric, etc. The topsheet materials should be soft and non-irritating to the skin and be readily penetrated by body fluid, e.g. urine or menstrual fluid. The topsheet may further be different in different parts of the absorbent article.

The backsheet 14 forms the outer cover of the absorbent article. The backsheet may be the same or different in different parts of the absorbent article. At least in the area of the absorbent structure 16, the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapour to escape from the absorbent core, while still preventing liquids from passing there through. Examples of breathable backsheet materials are porous polymeric films, nonwoven laminates of spunbond and meltblown layers and laminates of porous polymeric films and nonwoven materials. The backsheet may comprise a nonwoven material on at least the undergarment-facing surface thereof.

The absorbent structure 16 can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent core. Superabsorbent polymers are water-swellable, water-insoluble organic or inorganic materials capable of absorbing at least about 20 times their own weight of an aqueous solution containing 0.9 weight percent of sodium chloride. Organic materials suitable for use as superabsorbent materials can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fibre, flake, sphere, etc. possesses a higher crosslink density than the inner portion of the superabsorbent. The superabsorbent materials may be in any form which is suitable for use in absorbent composites including particles, fibres, flakes, spheres, and the like.

A high liquid storage capacity is provided by the use of high amounts of superabsorbent material. For an absorbent structure comprising a matrix of hydrophilic fibres, such as cellulosic fibres, and superabsorbent material, the proportion of superabsorbent material is preferably between 10 and 90% by weight, more preferably between 30 and 70% by weight.

It is conventional for absorbent articles to have absorbent structures comprising layers of different properties with respect to liquid receiving capacity, liquid distribution capacity and storage capacity. The absorbent structures, which are common in for example baby diapers and incontinence guards, often comprise a compressed, mixed or layered structure of cellulosic fluff pulp and superabsorbent polymers. The size and absorbent capacity of the absorbent core may be varied to suit different uses, such as infants or adult incontinent persons.

The absorbent structure 16 may further include an acquisition distribution layer placed on top of the primary absorbent body, which is adapted to quickly receive and temporarily store discharged liquid before it is absorbed by the primary absorbent core. Such acquisition distribution layers are well known in the art and may be composed of porous fibrous wadding or foam materials.

The first and second standing-gather webs 38, 40 may comprise or consist of any of the materials described above for the topsheet 66.

The first and second side panels 54, 56 may comprise or consist of an elastic web material such as an elastic film, an elastic nonwoven, an elastic laminate or the like. The elastic laminate may be a laminate between two or more nonwoven layers, two or more film layers or a combination of film and nonwoven layers.

The elastic material for the side panels 54, 56 may be constituted by an elastic laminate comprising at least one elastic film layer and at least one non-woven layer, in which the layers have been ultrasonically bonded, adhesively bonded or extrusion bonded, or bonded using a combination of the bonding methods. For such elastic laminates it is preferred that the first and second layers of fibrous material are chosen so that they, in combination with the intermediate elastic film layer, provide a soft and cloth-like feel to the laminate. Examples of suitable materials are carded webs and spun-bonded materials.

Examples of suitable polymers used in the fibrous materials are polyethylene, polyesters, polypropylene and other polyolefin homo-polymers and copolymers. Natural fibres, for example cotton, may also be used as long as they provide the required properties. A mixture of polymers can contribute to a higher flexibility of the non-woven layer.

In the event that the layers of the elastic laminate have been ultrasonically bonded, the male side of the bonding points should be arranged to be located towards the body of the user of the absorbent article when said absorbent article is in use. The male side of the bonding points is the non-woven surface comprising projections, such as projecting fibres, and the female side of said bonding points is the surface comprising depressions.

The elastic material for the side panels may comprise a stretch activated laminate, activated by hot stretching for example, and known in the art. The elastic laminate may be a laminate between two or more non-woven layers, two or more film layers or a combination of film and non-woven layers. One group of elastic laminates are so called "stretch-bonded" laminates, in which the elastic layer is stretched in at least one direction before laminating it with one or more inelastic layers. After the tension is removed from the elastic layer it can freely retract to its un-tensioned state, and the inelastic layer(s) laminated thereto become gathered, giving a three-dimensional puckering. Alternatively, the elastic material for side panels may comprise one or more elastic strips or threads contractably affixed between web materials, which may be inelastic.

Another group of elastic laminates are so called "neck bonded" laminates, which refer to laminates in which an elastic material is bonded to a non-elastic material while the non-elastic member is extended under conditions reducing its width or "necked". "Neck bonded laminate" refers to a composite material having at least two layers in which one layer is a necked, non-elastic layer and the other layer is an elastic layer. The layers are joined together when the non-elastic layer is in an extended condition.

A further group of elastic laminates are disclosed in for example WO 03/047488, in which inelastic non-woven layers are laminated to an elastic film layer, and the laminate is stretched above the point of failure of the non-woven materials, so that the inelastic layers break. Inelastic non-woven layers may also be laminated to an un-stretched elastic film layer. The elasticity of the laminate is then activated by mechanical stretching.

Examples of elastic laminates are described in EP-B-0 646 062, WO 98/29251, WO 03/000165 and US-A-5,226,992. Examples of commercially available elastic laminates are Fabriflex™ 306 from Tredegar and PK 6358 from Nordenia.

As used herein, an elastic material is defined as a material having a permanent elongation after relaxation of less than 10% after the material has been subjected to an elongation of 30% in the elasticity test outlined below. An elongation of 30% means an elongation to a length that is 30% longer than the initial length of the sample. An inelastic material has a permanent elongation after relaxation of more than 10% after having been subjected to an elongation of 30%.

### Elasticity test

The method measures the behaviour of an elastic material at repeated load and unload cycles. The sample is stretched to a predetermined elongation and a cyclic movement between 0 and said predetermined elongation is performed. Desired load and unload forces are recorded. The permanent, i.e. remaining, elongation of the relaxed material is measured.

The permanent elongation after relaxation should be less than 10% and is measured by the method below. Thus an elastic elongation of 30% is defined as that the laminate should have a permanent relaxation after elongation of less than 10% after being exerted to an elongation of 30% in the tensile tester below. An elongation of 30% means an elongation to a length that is 30% longer than the initial length of the sample.

A tensile tester, e.g. Lloyd LRX^{™}, able to perform cyclic movements and equipped with a printer/plotter or software presentation is used. The sample is prepared by cutting it to a width of 25 mm. The length and width of the sample may vary according to the available amount of material.

If the material to be tested has a width higher than 25 mm the sample should be cut so that the width is 25 mm. If however the material to be tested, e.g. the elastic material, has a width that is smaller than 25 mm the sample should have the width of the available material piece. The forces then have to be adjusted to the width of the sample according to the values given in brackets in the test method.

The tensile tester is calibrated according to the apparatus instructions. The parameters needed for the test (load and unload forces) are adjusted to:
- Crosshead speed: 500 mm/min
- Clamp distance: adapted to the length of the test sample
- Preload: 0.05 N (0.02 N/10mm width)

The sample is placed in the clamps and it is made sure that the sample is centred and fastened perpendicularly in the clamps. Depending on the length of the sample the distance between the clamps may vary. If a sample is longer than 100 mm it should be cut to a length of 100 mm. A suitable distance between the clamps is in this case 50 mm. For shorter samples the distance between the clamps can be shorter then 50 mm but as long as possible. For very short samples, less than 20 mm, the elastic sample should at both ends still be attached to inelastic material of the belt member or side panels, wherein the inelastic material is fastened in the clamps with the elastic part of the sample extending between the clamps. It is in this case important that the entire elastic part of such a sample is located between the clamps.

The tensile tester is started and two cycles between 0 and the predetermined elongation are performed. The crosshead should return immediately and not be held in the stretched position. There should not be any delay between the two cycles of the test method. After the last cycle, the sample is relaxed for 1 minute, then the permanent elongation is measured by stretching the sample until a force of 0.1 N (0.04 N/10mm width) is detected and the elongation is read.

In the illustrated embodiments, the leg elastic material 36 is depicted as being two strands of an elastic material, although a fewer or a greater number of strands can be utilized if desired. It has been found that two strands provide adequate elastic strength to form gathers about each of the leg openings. Materials suitable for forming the leg elastics 36 include polyurethane, rubber, elastic laminates and other elastomeric materials. The leg elastics can be in the form of long or short elastic strands, elastic tapes, ribbons, yarns, etc. The leg elastic material can have a cross-sectional configuration that is flat, square, rectangular, circular, oval or some other shape. A suitable material is LYCRA. LYCRA is a registered trademark of the E. I. DuPont de Nemours & Company that has an office at 1002 Market Street, Wilmington, Del. 19801.

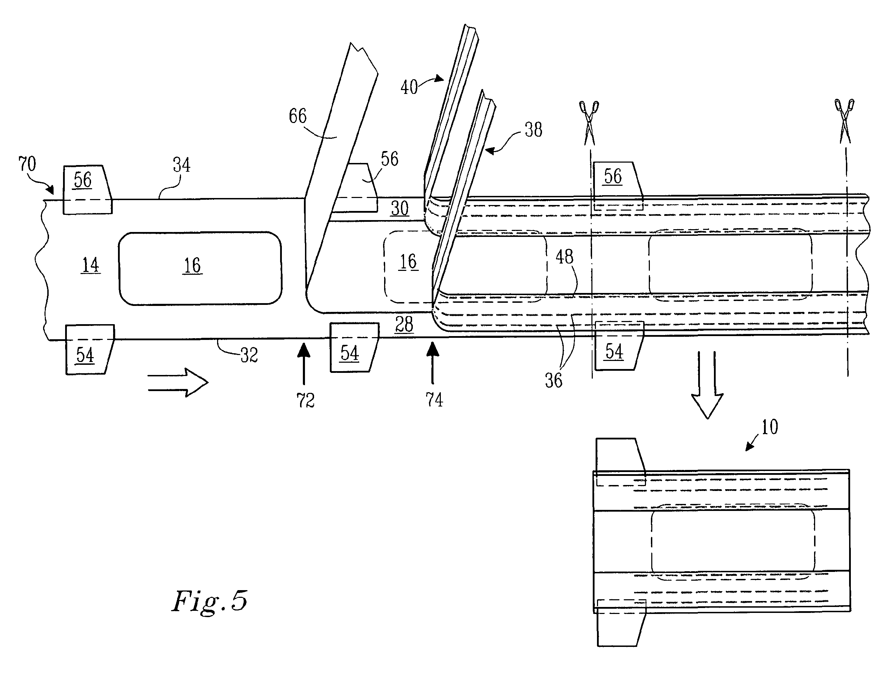

A method for manufacturing of the absorbent article 10 of the present invention will be described in the following with particular reference to Figs. 5 and 6. Thus, Fig. 6 is a schematic drawing of a part of the process to produce an absorbent article according to the invention. There are further process steps before and after this part of the process, but they will not be described in further detail since they are according to known technology. As far as possible, the components used in the manufacturing process will be denoted by the reference numbers used for the equivalent components in the absorbent article described above and illustrated in Figs. 1 to 4.

In Fig. 5, a continuous web 70 enters from the left-hand side. The process has a machine direction running towards the right-hand side of the drawing. The machine direction is parallel to the longitudinal axis L of the finished absorbent article. The web 70 comprises a backsheet 14, first and second side panels 54, 56 and an absorbent structure 16. The side panels 54, 56 extend in the cross machine direction from first and second lateral edges 32, 34 of the backsheet 14. The distance between the trailing edge (seen in the machine direction) of two consecutive side panel members along the same lateral side corresponds to the length H of the absorbent article. The length of absorbent structure 16 is normally shorter than the length H of the absorbent article.

At a first station, generally denoted by reference number 72, a topsheet 66 is applied to the web 70 to cover the absorbent structure 16. Additional (not shown) layers such as a liquid acquisition sheet may also be applied. In Fig. 5 the topsheet 66 is illustrated as being narrower than the backsheet 14, though it is to be understood that the topsheet and backsheet may be coextensive.

At a second station, generally denoted by reference number 74, first and second standing-gather webs 38, 40 are applied along each side of the web 1. Each standing-gather web comprises leg elastic material 36 and standing-gather elastic material 48.

Fig. 6 illustrates in more detail how one of the standing-gather webs 38 is prepared before being applied to the web 70. In Fig. 2 an embodiment is shown with two elastic yarns 36 for the leg elastic material and one elastic yarn 48 for the standing-gather elastic, though it is to be understood that the numbers of yarn may vary. The standing-gather web 38 is initially in a flat condition and the elastic yarns 36, 48 are applied under tension, that is in a stretched state. The direction of the yarns is essentially the same as the running direction of the standing-gather web 38. The yarns are intermittently adhered to the standing-gather web for example using an adhesive which is sprayed on the web via nozzles 76. The yarns could instead be attached by other means than adhesive, e.g. by ultrasonic bonding. After applying the yarns 36, 48 to the standing-gather web 38, the first end edge region 42 of the standing-gather web is folded over the standing-gather elastic yarn 48 to form the first pocket 46 and the second end edge region 44 is folded over the leg elastic yarns 36 to form the first leg elastic channel 50. The second standing-gather web 40 is prepared in an identical manner.

With reference again to Fig. 5, the thus-prepared standing-gather webs 38, 40 are applied and affixed to the web 70 in the first and second lateral margins 28, 30 such that the standing-gather elastic material 48 overlaps the topsheet 66. The first and second side panel members 54, 56 are partially overlapped by the standing-gather webs 38, 40 thereby creating the previously described first 60 and second 62 overlap regions, respectively. In the regions close to the transverse ends of the absorbent article, no standing-gather "pocket" is needed. As such, the standing-gather webs may be attached to the underlying topsheet 8 over the whole transverse extension of the standing-gather webs. This will help to keep the ends of the standing-gather webs in place on the absorbent article.

In a subsequent process step, the absorbent articles are separated from each other by cutting the web 70. The elastic yarns 36, 48 are also cut, thereby releasing the tension such that they contract. In the first and second overlap regions 60, 62 the ends of the leg elastic material are free to snap back in the leg elastic channels 50, 52. As such, these regions will not be gathered by the elastic material. In the areas where the elastic material is adhered to the channels, the elastic material will gather the absorbent article thereby imparting a bow-shaped form to the article.

The invention has been described above by way of example only and the skilled person will appreciate that many modifications of the above-described embodiments are conceivable within the scope of the appended claims. For example, although the absorbent article 10 has been illustrated as an open diaper which is intended to be closed when worn on an intended user, the invention is also applicable to a belted product or a pant diaper. For a belted product, the first and second side panels are constituted by belt halves. Furthermore, Figs. 1 to 4 illustrate embodiments with both leg elastic material and standing-gather elastic material. However, in its broadest form, the invention requires only leg elastic material. In addition, although the standing- gather webs have been illustrated as having an "L" shape, the invention is equally applicable to absorbent articles having "C"-shaped standing-gather webs.

## Claims

1. An absorbent article (10), comprising:
• a chassis (12) comprising a backsheet (14) and an absorbent structure (16), said chassis extending in a longitudinal direction about a longitudinal axis (L) dividing the chassis into a first lateral half (18) and a second lateral half (20), said chassis extending in a transverse direction about a transverse axis (T), said chassis being divided along the longitudinal axis into three regions,
a first end region (22) intended to be worn against the back of a user,
a crotch region (24) intended to be worn against the crotch of the user and
a second end region (26) intended to be worn against the stomach of the user,
said absorbent structure (16) being associated with said backsheet (14) such that said first lateral half (18) comprises a first lateral margin (28) terminating in a first lateral edge (32), and said second lateral half (20) comprises a second lateral margin (30) terminating in a second lateral edge (34), said first and said second lateral margins being free of said absorbent structure (16),
• leg elastic material (36) extending in the longitudinal direction in said first and second lateral margins (28, 30),
• a first and a second standing-gather web (38, 40) extending in the longitudinal direction affixed to said chassis in said first and second lateral margins, said first web (38) forming a first leg elastic channel (50) and said second web (40) forming a second leg elastic channel (52), said leg elastic channels accommodating said leg elastic material, and
• a first and a second side panel (54, 56) attached to the chassis in one of said first and second end regions (22, 26), said first side panel extending outwardly from said first lateral edge (32) of said chassis and said second side panel extending outwardly from said second lateral edge (34) of said chassis,
**characterised in that**
said first leg elastic channel (50) and said first side panel (54) are in an overlapping relationship in a first overlap region (60);
said second leg elastic channel (52) and said second side panel (56) are in an overlapping relationship in a second overlap region (62);
said leg elastic material (36) is at least partly adhered to said first and second standing-gather webs (38, 40) inside said first and second leg elastic channels (50, 52) at least in said crotch region (24), and
said leg elastic material (36) being free to move in the longitudinal direction relative to said standing-gather webs (38, 40) inside said leg elastic channels (50, 52) in said overlap regions (60, 62).

2. The absorbent article (10) according to claim 1, wherein said first and second side panels (54, 56) are attached to the first end region (22) of said chassis.

3. The absorbent article (10) according to claim 1 or 2, wherein each of said first and second side panels (54, 56) is fastened between said backsheet (14) and a topsheet (66).

4. The absorbent article (10) according to any one of claims 1 to 3, wherein a part of said first standing-gather web (38) contacts said first side panel (54) in said first overlap region (60) and a part of said second standing-gather web (40) contacts said second side panel (56) in said second overlap region (62).

5. The absorbent article (10) according to any of the preceding claims, wherein said leg elastic material (36) in said first leg elastic channel (50) extends over at least part of said first overlap region (60) and said leg elastic material in said second leg elastic channel (52) extends over at least part of said second overlap region (62).

6. The absorbent article (10) according to any of the preceding claims, wherein said leg elastic material (36) in said first and second leg elastic channels (50, 52) is free to move a free-moving length (D) which is 10-30% of the length (H) of the absorbent article, preferably 12-28% and most preferably 15-25%.

7. The absorbent article (10) according to any of the preceding claims, wherein the first and second lateral edges (32, 34) are straight.

8. The absorbent article according to claim 7, wherein said first and second lateral edges (32, 34) extend parallel to the longitudinal axis (L).

9. The absorbent article (10) according to any of the preceding claims, wherein said first and second side panels (54, 56) comprise an elastic material.

10. The absorbent article (10) according to any of the preceding claims, wherein each of said first and second standing-gather webs (38, 40) further comprises a standing-gather elastic (48) extending in the longitudinal direction and/or each of said first and second standing-gather webs (38, 40) further comprises a free-standing first end edge region (42) which extends freely from a proximal end (45) thereof, said proximal end (45) being located transversally inwardly of said leg elastic material (36).

11. The absorbent article (10) according to any of the preceding claims, wherein the maximal width A of the first lateral margin (28) is less than 65 mm, preferably less than 60 mm, more preferably less than 50 mm and most preferably less than 35 mm.

12. The absorbent article (10) according to any of the preceding claims, wherein the transverse distance C from the first lateral edge (32) to the first leg elastic channel (50) is less than 50 mm, preferably less than 40 mm, more preferably less than 30 mm and most preferably less than 20 mm.

13. The absorbent article (10) according any of the preceding claims, wherein said absorbent article forms an open diaper, which is intended to be closed when worn on an intended user, or said absorbent article forms a belted product, or said absorbent article forms a pant diaper.

14. A method of manufacturing an absorbent article (10), said method comprising the steps of:
- placing an absorbent structure (16) on a backsheet (14) such that said backsheet presents a first lateral margin (28) terminating in a first lateral edge (32) and a second lateral margin (30) terminating in a second lateral edge (34), said first and second lateral margins being free from the absorbent structure;
- attaching a first side panel (54) and a second side panel (56) to said backsheet (14) in said first and second lateral margins, respectively, such that said side panels extend outwardly from said lateral edges (32, 34);
- providing a first and a second standing-gather web (38, 40) having a first and a second leg elastic channel (50, 52), each leg elastic channel containing tensioned leg elastic material (36) intermittently affixed to said leg elastic channels;
- attaching said first and second standing-gather webs (38, 40) to said first and second lateral margins (28, 30) such that said first and second standing-gather webs partly overlap said first and second side panels (54, 56) in a first (60) and a second (62) overlap region, said leg elastic material being unattached to said first and second leg elastic channels in said first and second overlap regions, and
- releasing the tension in said leg elastic material (36) to allow free-moving ends of said leg elastic material to snap back along said first and second leg elastic channels.

15. The method as claimed in claim 14, wherein said first and second side panels are constituted by belt halves.

## Patentansprüche

1. Saugfähiger Artikel (10), mit:
einem Chassis (12) mit einer Rücklage (14) und einer saugfähigen Struktur (16), wobei der Chassis sich in einer Längsrichtung um eine Längsachse (L) erstreckt, die den Chassis in eine erste seitliche Hälfte (18) und eine zweite seitliche Hälfte (20) aufteilt, der Chassis sich in Querrichtung um eine Querachse (T) erstreckt und der Chassis entlang der Längsachse in drei Bereiche aufgeteilt ist,
einen ersten Endbereich (22), der vorgesehen ist, auf dem Rücken eines Benutzers getragen zu werden,
einen Schrittbereich (24), der vorgesehen ist, im Schritt des Benutzers getragen zu werden und
einem zweiten Endbereich (26), der vorgesehen ist, auf dem Bauch des Benutzers getragen zu werden,
wobei die saugfähige Struktur (16) so mit der Rücklage (14) zusammenhängt, dass die erste seitliche Hälfte (18) einen ersten seitlichen Rand (28) aufweist, der in einer ersten seitlichen Kante (32) endet und die zweite seitliche Hälfte (20) einen zweiten seitlichen Rand (30) aufweist, der in einer zweiten seitlichen Kante (34) endet, wobei der erste und der zweite seitliche Rand frei von der saugfähigen Struktur (16) sind,
einem elastischen Beinmaterial (36), das sich in dem ersten und zweiten seitlichen Rand (28, 30) in der Längsrichtung erstreckt,
einem ersten und einem zweiten stehenden Sammelgewebe (38, 40), das sich befestigt an dem Chassis in dem ersten und zweiten Seitenrand in der Längsrichtung erstreckt, wobei das erste Gewebe (38) einen ersten elastischen Beinkanal (50) ausbildet und das zweite Gewebe (40) einen zweiten elastischen Beinkanal (52) ausbildet und die elastischen Beinkanäle das elastische Beinmaterial aufnehmen, und
einem ersten und einem zweiten Seitenpanel (54, 56), die in einem des ersten und zweiten Endbereichs (22, 26) angebracht sind, wobei das erste Seitenpanel sich von der ersten seitlichen Kante (32) des Chassis nach außen erstreckt und das zweite Seitenpanel sich von der zweiten seitlichen Kante (34) des Chassis nach außen erstreckt,
**dadurch gekennzeichnet, dass**
der erste elastische Beinkanal (50) und das erste Seitenpanel (54) in einem ersten Überlappungsbereich (60) in einem überlappenden Verhältnis stehen;
der zweite elastische Beinkanal (52) und das zweite Seitenpanel (56) in einem zweiten Überlappungsbereich (62) in einem überlappenden Verhältnis stehen;
das elastische Beinmaterial (36) zumindest in dem Schrittbereich (24) innerhalb des ersten und zweiten elastischen Beinbereichs (50, 52) zumindest teilweise an dem ersten und zweiten stehenden Sammelgewebe (38, 40) haftet, und
das elastische Beinmaterial (36) frei ist, sich in der Längsrichtung in den Überlappungsbereichen (60, 62) innerhalb der elastischen Beinkanäle (50, 52) relativ zu dem stehenden Sammelgeweben (38, 40) zu bewegen.

2. Saugfähiger Artikel (10) nach Anspruch 1, bei dem das erste und zweite Seitenpanel (54, 56) an dem ersten Endbereich (22) des Chassis angebracht sind.

3. Saugfähiger Artikel (10) nach Anspruch 1 oder 2, bei dem das erste und zweite Seitenpanel (54, 56) jeweils zwischen der Rücklage (14) und einer Oberlage (66) befestigt sind.

4. Saugfähiger Artikel (10) nach einem der Ansprüche 1 bis 3, bei dem ein Teil des ersten stehenden Sammelgewebes (38) das erste Seitenpanel (54) in dem ersten Überlappungsbereich (60) berührt und ein Teil des zweiten stehenden Sammelgewebes (40) das zweite Seitenpanel (56) in dem zweiten Überlappungsbereich (62) berührt.

5. Saugfähiger Artikel (10) nach einem der vorstehenden Ansprüche, bei dem sich das elastische Beinmaterial (36) in dem ersten elastischen Beinkanal (50) über mindestens einen Teil des ersten Überlappungsbereichs (60) erstreckt und sich das elastische Beinmaterial in dem zweiten elastischen Beinkanal (52) über zumindest einen Teil des zweiten Überlappungsbereichs (62) erstreckt.

6. Saugfähiger Artikel (10) nach einem der vorstehenden Ansprüche, bei dem das elastische Beinmaterial (36) in dem ersten und zweiten elastischen Beinkanal (50, 52) frei ist, sich eine Freibewegungslänge (D) zu bewegen, die 10-30 % der Länge (H) des saugfähigen Artikels, bevorzugt 12-28 % und am bevorzugtesten 15-25 % ist.

7. Saugfähiger Artikel (10) nach einem der vorstehenden Ansprüche, bei dem die erste und zweite seitliche Kante (32, 34) gerade sind.

8. Saugfähiger Artikel (10) nach Anspruch 7, bei dem sich die erste und zweite seitliche Kante (32, 34) parallel zu der Längsachse (L) erstrecken.

9. Saugfähiger Artikel (10) nach einem der vorstehenden Ansprüche, bei dem das erste und zweite Seitenpanel (54, 56) ein elastisches Material aufweist.

10. Saugfähiger Artikel (10) nach einem der vorstehenden Ansprüche, bei dem das erste und zweite stehende Sammelgewebe (38, 40) des Weiteren eine stehende Sammelelastik (48) aufweist, die sich in der Längsrichtung erstreckt, und/oder das erste und zweite stehende Sammelgewebe (38, 40) des Weiteren jeweils einen freistehenden ersten Endkantenbereich (42) aufweist, der sich frei von seinem proximalen Ende (45) erstreckt, wobei das proximale Ende (45) quer nach innen zu dem elastischen Beinmaterial (36) angeordnet ist.

11. Saugfähiger Artikel (10) nach einem der vorstehenden Ansprüche, bei dem die maximale Breite (A) des ersten seitlichen Rands (28) weniger als 65 mm, bevorzugt weniger als 60 mm, noch mehr bevorzugt weniger als 50 mm und am meisten bevorzugt weniger als 35 mm beträgt.

12. Saugfähiger Artikel (10) nach einem der vorstehenden Ansprüche, bei dem der Querabstand C von der ersten seitlichen Kante (32) zu dem ersten elastischen Beinkanal (50) weniger als 50 mm, bevorzugt weniger als 40 mm, noch bevorzugter weniger als 30 mm und am meisten bevorzugt weniger als 20 mm beträgt.

13. Saugfähiger Artikel (10) nach einem der vorstehenden Ansprüche, bei dem der saugfähige Artikel eine offene Windel ausbildet, die vorgesehen ist, geschlossen zu werden, wenn sie an einem bestimmten Benutzer getragen wird, oder der saugfähige Artikel bildet ein Gürtelprodukt aus oder der saugfähige Artikel bildet eine Hosenwindel aus.

14. Verfahren zum Herstellen eines saugfähigen Artikels (10), mit den Schritten:
- Platzieren einer saugfähigen Struktur (16) auf einer Rücklage (14), sodass die Rücklage einen ersten seitlichen Rand (28) darstellt, der in einer ersten seitlichen Kante (32) endet, und einen zweiten seitlichen Rand (30), der in einer zweiten seitlichen Kante (34) endet, wobei der erste und zweite seitliche Rand frei von der saugfähigen Struktur sind;
- Anbringen eines ersten Seitenpanels (54) und eines zweiten Seitenpanels (56) an der Rücklage (14) in dem ersten beziehungsweise zweiten Rand, sodass die Seitenpanels sich von dem seitlichen Kanten (32, 34) nach außen erstrecken;
- Bereitstellen eines ersten und zweiten stehenden Sammelgewebes (38, 40), die einen ersten und einen zweiten elastischen Beinkanal (50, 52) aufweisen, wobei jeder elastische Beinkanal vorgespanntes elastisches Beinmaterial (36) enthält, das mit Unterbrechungen an den elastischen Beinkanälen befestigt ist;
- Anbringen des ersten und zweiten stehenden Sammelgewebes (38, 40) an dem ersten und zweiten seitlichen Rand (28, 30), sodass das erste und zweite stehende Sammelgewebe das erste und zweite Seitenpanel (54, 56) in einem ersten (60) und einem zweiten (62) Überlappungsbereich teilweise überlappen, wobei das elastische Beinmaterial nicht an dem ersten und zweiten elastischen Beinkanal in dem ersten und zweiten Überlappungsbereich angebracht ist, und
- Freigeben der Spannung in dem elastischen Beinmaterial (36), um es sich freibewegenden Enden des elastischen Beinmaterials zu ermöglichen, entlang des ersten und zweiten elastischen Beinkanals zurückzuschnappen.

15. Verfahren nach Anspruch 14, bei dem das erste und zweite Seitenpanel durch Gürtelhälften ausgebildet sind.

## Revendications

1. Article absorbant (10) comprenant :
un support (12) comportant une feuille arrière (14) et une structure absorbante (16),
ledit support s'étendant dans une direction longitudinale autour d'un axe longitudinal (L) qui divise le support en une première moitié transversale (18) et en une deuxième moitié transversale (20), ledit support s'étendant dans une direction transversale autour d'un axe transversal (T), ledit support étant divisé en trois zones le long de l'axe longitudinal,
une première zone d'extrémité (22) destinée à être portée contre le dos d'un utilisateur,
une zone d'entrejambe (24) destinée à être portée contre l'entrejambe de l'utilisateur et
une deuxième zone d'extrémité (26) destinée à être portée contre le ventre de l'utilisateur,
ladite structure absorbante (16) étant associée à ladite feuille arrière (14) de telle manière que ladite première moitié transversale (18) comprend une première marge latérale (28) se terminant par un premier bord latéral (32) et que ladite deuxième moitié transversale (20) comprend une deuxième marge latérale (30) se terminant par un deuxième bord latéral (34), lesdites première et deuxième marges latérales étant exemptes de ladite structure absorbante (16),
- un matériau élastique (36) pour les jambes s'étendant dans la direction longitudinale dans les première et deuxième marges latérales (28, 30),
- une première et une deuxième bandes de fronçage (38, 40) s'étendant dans la direction longitudinale, fixées audit support dans les première et deuxième marges latérale, ladite première bande (38) constituant un premier canal élastique (50) pour la jambe et ladite deuxième bande (40) constituant un deuxième canal élastique (52) pour la jambe, lesdits canaux élastiques pour la jambe comportant ledit matériau élastique pour les jambes et
- un premier et un deuxième panneaux latéraux (54, 56) fixés au support dans l'une desdites première et deuxième zones d'extrémité (22, 26), ledit premier panneau latéral s'étendant vers l'extérieur à partir dudit premier bord latéral (32) dudit support et ledit deuxième panneau latéral s'étendant vers l'extérieur à partir dudit deuxième bord latéral (34) dudit support,
**caractérisé en ce que**
ledit premier canal élastique (50) pour la jambe et le premier panneau latéral (54) sont en relation de chevauchement dans une première zone de chevauchement (60) ;
ledit deuxième canal élastique (52) pour la jambe et ledit deuxième panneau latéral (56) sont en relation de chevauchement dans une deuxième zone de chevauchement (62) ;
**en ce que** ledit matériau élastique (36) pour les jambes adhérent au moins partiellement auxdites première et deuxième bandes de fronçage (38, 40) situées à l'intérieur desdits premier et deuxième canaux élastiques (50, 52) pour les jambes, au moins dans ladite zone d'entrejambe (24) ; et
ledit matériau élastique (36) pour les jambes étant libre de se déplacer dans la direction longitudinale par rapport auxdites bandes de fronçage (38, 40) situées à l'intérieur desdits canaux élastiques (50, 52) pour les jambes dans lesdites zones de chevauchement (60, 62).

2. Article absorbant (10) selon la revendication 1 dans lequel lesdits premier et deuxième panneaux latéraux (54, 56) sont fixés à ladite première zone d'extrémité (22) dudit support.

3. Article absorbant (10) selon la revendication 1 ou 2 dans lequel chacun desdits premier et deuxième panneaux latéraux (54, 56) est fixé entre ladite feuille arrière (14) et une feuille supérieure (66).

4. Article absorbant (10) selon l'une quelconque des revendications 1 à 3 dans lequel une partie de ladite première bande de fronçage (38) est en contact avec ledit premier panneau latéral (54) dans ladite première zone de chevauchement (60) et dans lequel une partie de ladite deuxième bande de fronçage (40) est en contact avec ledit deuxième panneau latéral (56) dans ladite deuxième zone de chevauchement -62).

5. Article absorbant (10) selon l'une quelconque des revendications qui précèdent dans lequel ledit matériau élastique (36) pour les jambes présent dans ledit premier canal élastique (50) pour la jambe s'étend sur au moins une partie de ladite première zone de chevauchement (60) et dans lequel ledit matériau élastique pour les jambes présent dans ledit deuxième canal élastique (52) pour la jambe, s'étend sur au moins une partie de ladite deuxième zone de chevauchement (62).

6. Article absorbant (10) selon l'une quelconque des revendications qui précèdent dans lequel ledit matériau élastique (36) pour les jambes présent dans lesdits premier et deuxième canaux élastiques (50, 52) pour les jambes, est libre de se déplacer sur une distance de déplacement libre (D) dont la longueur est de 10 à 30 % de la longueur (H) de l'article absorbant, de préférence de 12 à 28 % et le plus préférablement de 15 à 25 %.

7. Article absorbant (10) selon l'une quelconque des revendications qui précèdent dans lequel les premier et deuxième bords latéraux (32, 34) sont droits.

8. Article absorbant selon la revendication 7 dans lequel lesdits premier et deuxième bords latéraux (32, 34) s'étendent parallèlement à l'axe longitudinal (L).

9. Article absorbant (10) selon l'une quelconque des revendications qui précèdent dans lequel lesdits premier et deuxième panneaux latéraux (54, 56) comportent un matériau élastique.

10. Article absorbant (10) selon l'une quelconque des revendications qui précèdent dans lequel chacune desdites première et deuxième bandes de fronçage (38, 40) comprennent en outre un élastique de fronçage (48) s'étendant dans la direction longitudinale et/ou dans lequel chacune des première et deuxième bandes de fronçage (38, 40) comporte en outre une première zone de bord d'extrémité librement relevée (42) qui s'étend librement à partir d'une extrémité proximale (45) de celle-ci, ladite extrémité proximale (45) étant située transversalement vers l'intérieur dudit matériau élastique (36) pour la jambe.

11. Article absorbant (10) selon l'une quelconque des revendications qui précèdent dans lequel la largeur maximum (A) de la première marge latérale (28) est inférieure à 65 mm, de préférence inférieure à 60 mm, plus préférablement inférieure à 50 mm, et le plus préférablement inférieure à 35 mm.

12. Article absorbant (10) selon l'une quelconque des revendications qui précèdent dans lequel la distance transversale (C) du premier bord latéral (32) au premier canal élastique (50) pour la jambe est inférieure à 50 mm, de préférence inférieure à 40 mm, plus préférablement inférieure à 30 mm et le plus préférablement inférieure à 20 mm.

13. Article absorbant (10) selon l'une quelconque des revendications qui précèdent dans lequel ledit article absorbant forme une couche ouverte qui est destinée à être fermée lorsqu'elle est portée par un futur utilisateur, ou dans lequel ledit article absorbant forme un article comportant une ceinture, ou dans lequel ledit article absorbant constitue une couche-culotte.

14. Procédé de fabrication d'un article absorbant (10), ledit procédé comprenant les opérations constituant à :
- mettre en place une structure absorbante (16) sur une feuille arrière (14) de telle manière que ladite feuille arrière présente une première marge latéral (28) qui se termine par un premier bord latéral (32) et une deuxième marge latérale (30) qui se termine par un deuxième bord latéral (34), lesdites première et deuxième marges latérales étant exemptes de la structure absorbante ;
- fixer un premier panneau latéral (54) et un deuxième panneau latéral à ladite feuille arrière (14) respectivement dans une première et une deuxième marges latérales de telle manière que lesdits panneaux latéraux s'étendent vers l'extérieur à partir desdits bords latéraux (32, 34) ;
- ménager une première et une deuxième bandes de fronçage (38, 40) comportant un premier et un deuxième canal élastique (50, 52) pour les jambes, chaque canal élastique pour les jambes contenant un matériau élastique (36) pour la jambe mis sous tension et fixé par intermittence auxdits canaux élastiques pour les jambes ;
- fixer lesdites première et deuxième bandes de fronçage (38, 40) auxdites première et deuxième marges latérales (28, 30) de telle manière que lesdites première et deuxième bandes de fronçage chevauchent partiellement lesdits premier et deuxième panneaux latéraux (54, 56) dans une première zone de chevauchement (60) et dans une deuxième zone de chevauchement (62), ledit matériau élastique pour les jambes n'étant pas fixé auxdits premier et deuxième canaux élastiques pour les jambes dans lesdites première et deuxième zones de chevauchement, et
- relâcher la tension dans ledit matériau élastique (36) pour les jambes afin de permettre aux extrémités dudit matériau élastique pour les jambes libres de se déplacer de revenir brusquement le long desdits premier et deuxième canaux élastiques pour les jambes.

15. Procédé selon la revendication 14 dans lequel lesdits premier et deuxième panneaux latéraux sont constitués par des moitiés de ceinture.
